# EUROPEAN PATENT SPECIFICATION

(11) **EP 1 835 817 B1**
(45) Date of publication and mention of the grant of the patent: **14.02.2018**
(21) Application number: 05855955.0
(22) Date of filing: 30.12.2005
(51) Int. Cl.: A23K 10/20, A23K 10/30, A23K 20/163, A23K 50/48

(54) **COMPOSITIONS FOR FELINE CONSUMPTION**
ZUM VERZEHR DURCH KATZEN BESTIMMTE ZUSAMMENSETZUNGEN
COMPOSITIONS ALIMENTAIRES DESTINEES AUX FELINS

(30) Priority: 30.12.2004 US 640566 P
(43) Date of publication of application: 26.09.2007
(73) Proprietor: Hill's Pet Nutrition, Inc., Topeka, KS 66603 (US)
(72) Inventor: BOWMAN, Eric, Vaun, Topeka, KS 66614 (US); CLARK, Joseph, Robert, Topeka, KS 66614 (US); MADRIL, Michael, Timothy, Topeka, KS 66614 (US); CHEUK, Wai, Lun, San Marcos, CA 92078 (US); DIERKING, Mark, Lee, Topeka, KS 66618 (US)
(74) Representative: Wichmann, Hendrik
(86) International application number: PCT/US2005/047466
(87) International publication number: WO 2006/074091

(56) References cited:
- EP-A1- 0 337 027
- WO-A1-01/17366
- WO-A1-99/45794
- GB-A- 2 353 200
- US-A- 6 040 292
- US-A1- 2004 023 885
- US-B1- 6 203 825
- US-B1- 6 410 063
- US-B1- 6 436 463
- US-B2- 6 692 787

## Description

### CROSS-REFERENCE TO RELATED APPLICATIONS

This application claims priority to U.S. Provisional Application Serial No. 60/640,566, filed December 30, 2004.

### BACKGROUND OF THE INVENTION

### Field of the Invention

This invention relates generally to compositions for animal consumption and particularly to meat and carbohydrate based compositions for feline consumption.

### Description of the Related Art

Companion animals have been fed "dry" and "wet" food compositions for many years. "Wet" food compositions are generally packaged in can-like containers and are considered "wet" in appearance because of the moisture contained therein. Two types of wet compositions are generally known in the art. The first is known in the art as "ground loaf." Loaf products are typically prepared by contacting a mixture of components under heat to produce an essentially homogeneous, intracellular honeycomb-type mass or "ground loaf." The ground loaf mass is then packaged into a cylindrical container, such as a can. Upon packing, ground loaf assumes the shape of the container such that the ground loaf must be cut when serving to a companion animal. As a result of processing, ground loaf products exhibit a wide range of textural differences and loaf products generally do not mix well with other forms of foods, especially dry products.

Another type of wet composition is generally known in the art as "chunk and gravy." Chunk and gravy products comprise a preformed meat particle prepared by making a meat emulsion which is extruded and formed by physical pressure or thermal energy such as cooking with steam, cooking in water, oven dry heat and the like. A product, such as cooked meat, is diced into chunks, which are eventually mixed with a gravy or sauce. The two components are then filled into a container, usually a can, which is seamed and sterilized. As opposed to the ground loaf, chunk and gravy compositions have physically separated, discrete chunks (i.e., pieces of ground meat and grains) as prepared. These discrete particles are present in the gravy-type liquid in the final container. When serving, chunk and gravy products flow out of the can and can be easily mixed with other dry products. While the chunk and gravy products allow better integrity of the individual ingredients, the heterogeneous formulation of the chunk and gravy products are sometimes disfavored by consumers.

U.S. Patent No. 6,436,463 describes a third type of wet composition that is a "hybrid" of the two distinct physical forms of ground loaf and chunk and gravy compositions. This hybrid composition has the appearance of fine ground hamburger or hash with visually recognizable discrete meat particles within an essentially homogeneous mass of the finished product. The reference discusses a process for preparing such hybrid compositions wherein the process includes a first thermal process to preserve the physical and chemical integrity of the meat component and a modified-gravy process which serves to bind the meat and grain components. However, the hybrid compositions described in the reference typically contain less than about 70% by weight meat and have a non-fat carbohydrate equivalent of from about 25% to about 40% by weight as measured on a dry matter basis. Although these compositions are useful, there exists a need for compositions for feline consumption having a high meat concentration with a low non-fat carbohydrate equivalent.

### SUMMARY OF THE INVENTION

The present description provides compositions for feline consumption comprising at least about 65% by weight meat and having an essentially solid mass that assumes the shape of its container. In one aspect, the composition further comprises carbohydrate in an amount of less than about 10% by weight of the composition. In another aspect, the composition comprises at least about 70% by weight meat and less than about 10% by weight grain, preferably from about 2% to about 10% by weight of a grain mixture comprising one or more grains. The compositions have a moisturized appearance and visually recognizable discrete food particles upon slicing the mass after removal from a container.

In another aspect, the description provides methods for treating or managing feline diabetes. The method comprises feeding a composition of the present invention to a feline susceptible to or suffering from diabetes.

The present invention provides a process for preparing a meat and carbohydrate based feline pet food composition having an essentially homogeneous mass substantially conforming to the shape of its container and the product produced by the process. The process comprises contacting a meat component, a grain component, and a heated gravy component for a time and at a temperature sufficient to prepare an essentially homogeneous mass, wherein the composition comprises at least about 65% by weight meat and wherein the meat component is not heated prior to contacting the heated gravy. In a particular embodiment, the temperature of the meat component and the grain component is less than about 40°C prior to contacting the heated gravy.

Other and further objects, features, and advantages of the present invention will be readily apparent to those skilled in the art.

### DETAILED DESCRIPTION OF THE INVENTION

### Definitions

The term "diabetes drug" means any compound, composition, or drug useful for preventing or treating feline diabetes.

The term "food" means not only a food product which typically provides most, if not all, the nutrient value for a companion animal, but may also items as a snacks, treats, supplements, food toys, and the like.

The term "single package" means that the components of a kit are physically associated in or with one or more containers and considered a unit for manufacture, distribution, sale, or use. Containers include, but are not limited to, bags, boxes, bottles, shrink wrap packages, stapled or otherwise affixed components, or combinations thereof. A single package may be containers of individual food compositions and diabetes drugs physically associated such that they are considered a unit for manufacture, distribution, sale, or use.

The term "virtual package" means that the components of a kit are associated by directions on one or more physical or virtual kit components instructing the user how to obtain the other components, e.g., in a bag containing one component and directions instructing the user to go to a website, contact a recorded message, view a visual message, or contact a caregiver or instructor to obtain instructions on how to use the kit.

Unless otherwise specified, the term "by weight" refers to weight percentage of the total composition calculated on a dry weight basis.

This invention is not limited to the particular methodology, protocols, and reagents described herein because they may vary. Further, the terminology used herein is for the purpose of describing particular embodiments only and is not intended to limit the scope of the present invention. As used herein and in the appended claims, the singular forms "a," "an," and "the" include plural reference unless the context clearly dictates otherwise. Similarly, the words "comprise", "comprises", and "comprising" are to be interpreted inclusively rather than exclusively.

Unless defined otherwise, all technical and scientific terms and any acronyms used herein have the same meanings as commonly understood by one of ordinary skill in the art in the field of the invention. Although any methods and materials similar or equivalent to those described herein can be used in the practice of the present invention, the preferred methods, devices, and materials are described herein.

All patents, patent applications, and publications mentioned herein are incorporated herein by reference to the extent allowed by law for the purpose of describing and disclosing the compounds, processes, techniques, procedures, technology, articles, and other compositions and methods disclosed therein that might be used with the present invention. However, nothing herein is to be construed as an admission that the invention is not entitled to antedate such disclosure by virtue of prior invention.

### The Invention

The invention provides a process for preparing a meat and carbohydrate based feline food composition consisting essentially of a solid and homogeneous mass that substantially conforms to the shape of its container comprising contacting a meat component, a grain component, and a heated gravy component for a time and at a temperature sufficient to prepare a food composition consisting essentially of a solid and homogeneous mass, wherein the composition comprises at least 65% by weight meat, and wherein the meat component is not heated prior to contacting the heated gravy.

The description provides a meat composition for feline consumption comprising at least about 65% by weight meat. The composition is an essentially homogeneous mass substantially conforming to the shape of its container. The compositions have visually recognizable discrete meat particles with a moisturized appearance upon slicing the solid mass after departure from a container. In various embodiments, the "high meat" compositions of the present invention comprise:

| Component | |
|---|---|
| Meat | from about 65% by weight to about 80% by weight (or from about 70% by weight to about 80% by weight) |
| Grains | from about 2% by weight to about 15% by weight (or from about 2% by weight to about 10% by weight) |
| Vitamins, Minerals, | from about 0% by weight to about 5% by weight (or from |
| Colorant, Flavor | about 1% by weight to about 3% by weight) |
| Gravy | from about 15% by weight to about 40% by weight (or from about 15% by weight to about 30% by weight) |

In one aspect, the composition further comprises carbohydrate in an amount of less than about 10% by weight of the composition. In another, a high meat composition comprises at least about 70% by weight meat and less than about 10% by weight grain, preferably from about 2% to about 10% by weight of a grain mixture comprising one or more grains. In a further, a high meat composition prepared by the process of the present invention comprises at least about 75% by weight meat and less than about 25% by weight gravy.

The high meat compositions prepared by the process of the present invention are also advantageous in that they have a low non-fiber carbohydrate equivalent fraction. As used herein, the term "non-fiber carbohydrate equivalent fraction" or "NFE" of a composition is calculated as the remainder of a composition after subtracting the percentage of crude protein, crude fat, crude fiber and crude ash as calculated by weight on a dry matter basis. In particular, Applicants have discovered that the high meat compositions prepared by the process of the present invention generally have a non-fiber carbohydrate equivalent fraction of less than 15% by weight. In a particular embodiment, a high meat composition prepared by the process of the present invention comprises at least about 70% by weight meat and has a non-fiber carbohydrate equivalent fraction of less than about 10% by weight.

As described above, the processes of the present invention comprise contacting a meat component and a grain component with heated gravy for a time and at a temperature sufficient to prepare an essentially homogeneous mass. The processes are advantageous over known processes for preparing wet diet compositions that typically require each of the meat and grain components to be heated before mixing. Without being held to a particular theory, it is believed that sufficient heat for preparing a composition having an essentially homogeneous mass can be provided by the hot gravy component without separately heating the meat or grain components before mixing. As such, the present invention provides a process that is easier to operate and control and that results in lower operating costs and requires less capital equipment.

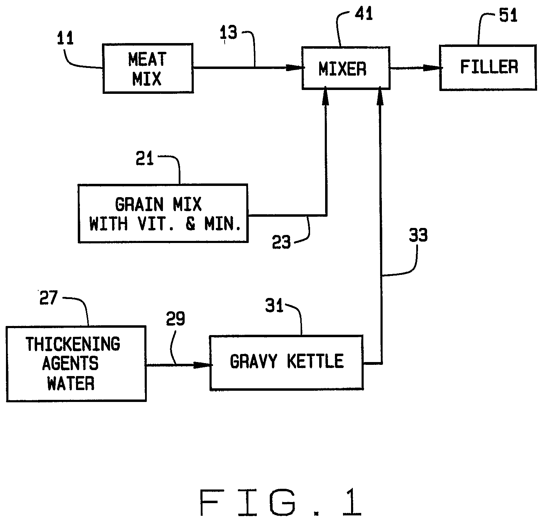

Referring now to Figure 1, the novel process of the present invention is more fully described. The process comprises preparing a meat mixture or a meat component in a mixer 11. The meat mixture is transferred through line 13 to a second mixer 41. A grain mixture comprising one or more grains (i.e., a grain component) is prepared in a mixer 21 and transferred through line 23 to second mixer 41. Heated gravy is prepared separately from the meat mixture and the carbohydrate mixture by contacting water and one or more thickening agents under heat in a gravy kettle 31. The heated gravy is then transferred through line 33 to second mixer 41. The meat mixture, grain mixture and heated gravy are all mixed together in second mixer 41 for a time and at a temperature sufficient to result in an essentially homogeneous mass. Additional heat can be added to maintain the prevailing temperature in the second mixer, if desired, but it is generally not necessary. The essentially homogeneous mass produced in second mixer 41 is then further processed for partitioning into containers, for example, using a filler 51. In this manner, the diet of this disclosure is readily prepared.

The meat mixture can be prepared from a wide variety of meats or meat sources including, for example, meat sources selected from the group consisting of animal muscle, animal skeletal meat, animal by-products, and mixtures of muscle, skeletal meat and by-products. Meats include, for example, the flesh of poultry; fish; and mammals (e.g., cattle, swine, sheep, goats, and the like). Meat by-products include, for example, lungs, kidneys, livers, tongues, stomachs and intestines. Suitable meat sources may include fresh and frozen meats or meat by-products. The meat mixture is generally prepared by grinding the meat through different grind-plates, typically ranging from about 1/2 inch to about 1 inch in size, to form the discrete food particles required for the finished product.

The prepared meat mixture or meat component for inclusion in the composition generally comprises at least about 15% by weight protein and about 25% by weight fat. For example, in one embodiment, the meat mixture comprises one or more animal protein sources such that the mixture comprises from about 15% to about 25% protein, from about 5% to about 15% by weight fat, and from about 55% to about 75% by weight water.

The meat mixture may be prepared in any suitable mixing apparatus known to one skilled in the art. Non-limiting examples of suitable apparatus for preparing the meat component include a twin screw mixer, a twin ribbon mixer, an overlapping paddle mixer, or a combination mixer such as a screw/ribbon/paddle.

The grain component comprises a mixture of one or more grains. Suitable grains include, for example, grains selected from the group consisting of oat fiber, cellulose, peanut hull, beet pulp, parboiled rice, corn starch, corn gluten meal and mixtures thereof. It is important to note that by properly balancing carbohydrate sources, one skilled in the art can manipulate the texture of the final product. For example, short chain polysaccharides tend to be sticky and gluey and longer chain polysaccharides are less sticky and gluey than the shorter chain. Basically the desired texture of this hybrid diet is achieved by longer chain polysaccharide and modified starches such as native or modified starches, cellulose and the like.

The grain mixture may additionally comprise optional components such as added salt, spices, seasonings, vitamins, minerals, flavorants, colorants, and the like. The amount of the optional additives is at least partially dependent on the nutritional requirements for different life stages of animals. For example, the National Research Council (NRC) provides recommended amounts of such ingredients for farm animals. See, e.g., Nutrient Requirements of Swine (10th Rev. Ed., Nat'l Academy Press, Wash. D.C., 1998), Nutrient Requirements of Poultry (9th Rev. Ed., Nat'l Academy Press, Wash. D.C., 1994), Nutrient Requirements of Horses (5th Rev. Ed., Nat'l Academy Press, Wash. D.C., 1989), etc. and the American Feed Control Officials (AAFCO), for example, provides recommended amounts of such ingredients for dogs and cats. See American Feed Control Officials, Inc., Official publication, pp. 126-140 (2003).

Vitamins generally useful as food additives include, for example, vitamin A, vitamin B1, vitamin B2, vitamin B6, vitamin B12, vitamin C, vitamin D, vitamin E, vitamin H (biotin), vitamin K, folic acid, inositol, niacin, and pantothenic acid. Minerals and trace elements generally useful as food additives include, for example, calcium, phosphorus, sodium, potassium, magnesium, copper, zinc, choline, and iron salts.

The gravy component is prepared by contacting one or more thickening agents with water at a temperature of from about 65° to about 85°C (or from about 70° to about 75°C). Thickening agents for use in the gravy component may include gums such as guar gum(s), native starch and various modified starches such as dextrin prepared from dry starch treated with heat and acid, oxidation of native starch with hypochlorite to obtain an oxidized starch which can inhibit gelling, an acid or enzyme hydrolyzed polysaccharide which can delay pasting and gelling, a crosslinked starch and a substituted starch with, for example, propylene oxide or acetic anhydride which can provide freeze thaw stability and a peak viscosity as well as a high fructose corn syrup, carbohydrates, glucose and sucrose. Therefore, a multitude of desirable results such as shelf life stability, process control, textural and mouth feel, heat acid stability, shear stability, and freeze thaw stability can be achieved through proper selection of the modification.

Heating of the gravy mixture may be effected using any suitable manner, such as, for example, by direct steam injection or by using a vessel fitted with a heat exchanger.

The gravy component generally comprises materials selected to provide a suitable viscosity when the meat component, grain component and gravy component are mixed together. The pre-processing viscosity is important to provide the essentially homogeneous mass and to prevent component separation when the composition is partitioned into containers (i.e., during filling). Thus it is important that the three components remain homogeneous at the filling stage.

The meat component, the grain component, and the hot gravy are mixed at temperature for a time sufficient to achieve or essentially achieve hydration and gelatinization of carbohydrates so as to improve the finished product texture. As described above, the process of the present invention does not require heating of the meat component or the grain component prior to contacting the heated gravy. Thus, the meat component and the grain component are typically prepared at a temperature of less than about 40°C, less than about 25°C or less than about 10°C. For example, in one embodiment, the meat component comprises a meat slurry that has a temperature of from about 0°C to about 5°C before being contacted with the heated gravy component.

The meat component, grain component, and heated gravy component are contacted in any suitable mixer. Examples of suitable mixing apparatus include a twin screw mixer, a twin ribbon mixer, or an overlapping paddle mixer. The mixing should be vigorous enough to ensure that the individual components are formed into a single entity for further processing and partitioning into containers. The temperature during mixing of the meat component, grain component and heated gravy is from about 25° to about 45°C to achieve or essentially achieve hydration and gelatinization of grain/carbohydrate for certain desired texture of diet. Additional heat may be provided, however, such is not necessary.

The final mixture is filled into cans which are then sealed and sterilized. In this case, the product produced a solid mass with recognizable discrete meat particles with a moisturized appearance. Additional water may also be added to the mixture until an essentially uniform, colloidal mass is produced having the desired texture. The final product is then placed in containers, seamed, and retorted for sterility.

The compositions prepared by the process of the present invention are suitable for use in preventing or treating diabetes in a feline susceptible to or suffering from diabetes. For example, the compositions prepared by the process of the present invention comprising at least about 70% by weight meat and having a non-fiber carbohydrate fraction of less than about 15% by weight as measured on a dry matter basis are fed to felines to manage glycemic control in cats predisposed to diabetes.

In a further aspect, the present description provides kits for preventing or treating diabetes. The kits comprise in separate containers in a single package or in separate containers in a virtual package, as appropriate for the kit component, a composition for feline consumption prepared by the process of the present invention and one or more of (1) one or more diabetes drugs and (2) instructions for how to use compositions prepared by the process of the present invention alone, or in combination with one or more diabetes drugs, to prevent or treat diabetes. When the kit comprises a virtual package, the kit is limited to instructions in a virtual environment in combination with one or more physical kit components. The kit contains the composition and other components in amounts sufficient to prevent or treat diabetes. Typically, composition, diabetes drugs, and/or other suitable kit components are admixed just prior to consumption by an animal. The kits may contain the kit components in any of various combinations and/or mixtures. In one aspect, the kit contains a packet containing one or more diabetes drugs and a container of food for consumption by an animal. The kit may contain additional items such as a device for mixing the kit components or a device for containing the admixture, e.g., a food bowl. In another aspect, composition and/or diabetes drugs are mixed with additional nutritional supplements such as vitamins and minerals that promote good health in an animal.

In another aspect, the present description provides a means for communicating information about or instructions for one or more of (1) using the compositions prepared by the process of the present invention for preventing or treating diabetes, (2) admixing the compositions with the other components of the present invention, (3) administering the compositions to a feline, alone or in combination with the other elements of the present invention, and (4) conducting the processes of the present invention. The means comprises a document, digital storage media, optical storage media, audio presentation, or visual display containing the information or instructions. In certain aspects, the communicating means comprises a document, digital storage media, optical storage media, audio presentation, or visual display containing the information or instructions. Preferably, the communication means is a displayed web site or a brochure, product label, package insert, advertisement, or visual display containing such information or instructions. Useful information includes one or more of (1) methods and techniques for combining and administering the compositions and/or other components and (2) contact information for animal caregivers to use if they have a question about the invention and its use. Useful instructions include amounts for mixing and administration amounts and frequency. The communication means is useful for instructing on the benefits of using the present invention and communicating the approved methods for administering the invention to an animal.

In a further aspect, the present description provides a use of a composition prepared by the process of the present invention to prepare a medicament. In another, the description provides for the use of such composition to prepare a medicament for preventing or treating diabetes in a feline susceptible to or suffering from diabetes. Generally, medicaments are prepared by admixing a compound or composition with excipients, buffers, binders, plasticizers, colorants, diluents, compressing agents, lubricants, flavorants, moistening agents, and other ingredients known to skilled artisans to be useful for producing medicaments and formulating medicaments that are suitable for administration to an animal.

### EXAMPLES

The invention can be further illustrated by the following examples of preferred embodiments thereof, although it will be understood that these examples are included merely for purposes of illustration and are not intended to limit the scope of the invention unless otherwise specifically indicated.

### Example 1

This example demonstrates the preparation of a high meat composition for feline consumption using the process of the present invention.

A meat mix comprising skeletal muscle from fish or chicken and meat by products was prepared by mixing the meat sources in a ribbon/paddle mixer.

A grain mixture was prepared in a separate mixture by contacting the grains with vitamins, minerals, colorant and flavor.

A hot gravy was then prepared by mixing together modified corn starch and guar gum with water/steam making up the remainder. The gravy was then heated to a temperature of from about 87°C (190°F) to about 93°C (200°F) until the mixture developed a target viscosity of about 20 cm / 30 seconds at 82°C (180°F) as measured on a Botswick Consistometer.

The meat mixture, grain mixture, and gravy were combined in a regular mixer and evenly blended without further heating. The blending ratio of meat mixture, grain mixture and gravy was 75:2.5:18 wt %, respectively. The resultant product after sterilization had a hearty ground texture appearance with visually recognizable meat particles which neither resemble ground loaf nor chunk and gravy products. The composition of the final product is shown in Table 1.

**Table 1**

| Component | % by weight of composition |
|---|---|
| Meat | 75 |
| Grain | 2.5 |
| Gravy | 18 |
| Protein | 13 |
| Fat | 8.5 |

In the specification, there have been disclosed typical preferred embodiments of the invention and, although specific terms are employed, they are used in a generic and descriptive sense only and not for purposes of limitation, the scope of the invention being set forth in the following claims. Obviously many modifications and variations of the present invention are possible in light of the above teachings. It is therefore to be understood that within the scope of the appended claims the invention may be practiced otherwise than as specifically described.

## Claims

1. A process for preparing a meat and carbohydrate based feline food composition consisting essentially of a solid and homogeneous mass that substantially conforms to the shape of its container comprising contacting a meat component, a grain component, and a heated gravy component for a time and at a temperature sufficient to prepare a food composition consisting essentially of a solid and homogeneous mass, wherein the composition comprises at least 65% by weight meat, and wherein the meat component is not heated prior to contacting the heated gravy.

2. The process of claim 1 wherein the heated gravy component is at a temperature of from 65° to 85°C before contacting the meat component or the grain component.

3. The process of claim 1 wherein the meat component comprises one or more meat sources selected from the group consisting of animal muscle, animal skeletal meat, animal by-products, and mixtures of muscle, skeletal meat and by-products.

4. The process of claim 1 wherein the grain component comprises one or more grains selected from the group consisting of oat fiber, cellulose, peanut hull, beet pulp, parboiled rice, cornstarch, corn gluten meal and mixtures thereof.

5. The process of claim 1 wherein the heated gravy comprises water and one or more thickening agents selected from the group consisting of corn starch, guar gum, glucose, sucrose, high fructose corn syrup and mixtures thereof.

6. The process of claim 1, wherein the composition comprises at least 75% by weight meat and non-fiber carbohydrate in an amount of less than 15% by weight of the composition.

7. The process of claim 6 comprising non-fiber carbohydrate in an amount of less than 10% by weight of the composition.

8. The process of claim 6 wherein the grain component comprises from 2% to 10% by weight of a grain mixture comprising one or more grains.

9. The process of claim 8 wherein the grain mixture comprises one or more grains selected from the group consisting of oat fiber, cellulose, peanut hull, beet pulp, parboiled rice, cornstarch, corn gluten meal and mixtures thereof.

10. The process of claim 1 comprising from 5% to 25% by weight of the heated gravy component.

11. The process of claim 10 wherein the heated gravy component comprises water and one or more thickening agents selected from the group consisting of corn starch, guar gum, glucose, sucrose, high fructose corn syrup and mixtures thereof.

12. The process of claim 1 wherein the meat comprises one or more meat sources selected from the group consisting of animal muscle; animal skeletal meat; animal byproducts; and mixtures of muscle, skeletal meat, and by-products.

13. The process of claim 1, wherein the temperature of the meat component is less than 25°C prior to contacting with the heated gravy.

## Patentansprüche

1. Verfahren zum Herstellen einer auf Fleisch und Kohlenhydrat basierenden Katzenfutterzusammensetzung, die im Wesentlichen aus einer festen und homogenen Masse besteht, die sich wesentlich an die Form ihres Behälters anpasst, umfassend Inkontaktbringen eines Fleischbestandteils, eines Getreidebestandteils und eines erhitzten Soßenbestandteils für eine Zeit und bei einer Temperatur, die ausreichend ist, eine Nahrungsmittelzusammensetzung herzustellen, die im Wesentlichen aus einer festen und homogenen Masse besteht, wobei die Zusammensetzung mindestens 65% bezogen auf das Gewicht an Fleisch umfasst, und wobei der Fleischbestandteil vor dem Inkontaktbringen mit der erhitzten Soße nicht erhitzt wird.

2. Verfahren nach Anspruch 1, wobei der erhitzte Soßenbestandteil sich bei einer Temperatur von 65° bis 85°C vor dem Inkontaktbringen mit dem Fleischbestandteil oder dem Getreidebestandteil befindet.

3. Verfahren nach Anspruch 1, wobei der Fleischbestandteil ein oder mehrere Fleischquellen umfasst, die aus der Gruppe bestehend aus Tiermuskel, Tierknochenfleisch, Tiernebenprodukten und Gemischen von Muskel, Knochenfleisch und Nebenprodukten ausgewählt sind.

4. Verfahren nach Anspruch 1, wobei der Getreidebestandteil ein oder mehrere Getreide umfasst, die aus der Gruppe bestehend aus Haferfaser, Cellulose, Erdnussschale, Rübenschnitzel, Parboiled-Reis, Maisstärke, Maisglutengrieß und Gemischen davon ausgewählt sind.

5. Verfahren nach Anspruch 1, wobei die erhitzte Soße Wasser und eines oder mehrere Verdickungsmittel umfasst, die aus der Gruppe bestehend aus Maisstärke, Guargummi, Glucose, Saccharose, Maissirup mit hohem Fructosegehalt und Gemischen davon ausgewählt sind.

6. Verfahren nach Anspruch 1, wobei die Zusammensetzung mindestens 75% bezogen auf das Gewicht an Fleisch und Nichtfaser-Kohlenhydrat in einer Menge von weniger als 15% bezogen auf das Gewicht der Zusammensetzung umfasst.

7. Verfahren nach Anspruch 6, das Nichtfaser-Kohlenhydrat in einer Menge von weniger als 10% bezogen auf das Gewicht der Zusammensetzung umfasst.

8. Verfahren nach Anspruch 6, wobei der Getreidebestandteil von 2% bis 10% bezogen auf das Gewicht einer Getreidemischung umfasst, die ein oder mehrere Getreide umfasst.

9. Verfahren nach Anspruch 8, wobei das Getreidegemisch ein oder mehrere Getreide umfasst, die aus der Gruppe bestehend aus Haferfaser, Cellulose, Erdnussschale, Rübenschnitzel, Parboiled-Reis, Maisstärke, Maisglutengrieß und Gemischen davon ausgewählt sind.

10. Verfahren nach Anspruch 1, das von 5% bis 25% bezogen auf das Gewicht erhitzten Soßenbestandteil umfasst.

11. Verfahren nach Anspruch 10, wobei der erhitzte Soßenbestandteil Wasser und ein oder mehrere Verdickungsmittel umfasst, die aus der Gruppe bestehend aus Maisstärke, Guargummi, Glucose, Saccharose, Maissirup mit hohem Fructosegehalt und Gemischen davon ausgewählt sind.

12. Verfahren nach Anspruch 1, wobei das Fleisch ein oder mehrere Fleischquellen umfasst, die aus der Gruppe bestehend aus Tiermuskel; Tierknochenfleisch; Tiernebenprodukten; und Gemischen von Muskel, Knochenfleisch und Nebenprodukten ausgewählt sind.

13. Verfahren nach Anspruch 1, wobei die Temperatur des Fleischbestandteils weniger als 25°C vor dem Inkontaktbringen mit der erhitzten Soße beträgt.

## Revendications

1. Procédé de préparation d'une composition alimentaire pour félin à base de viande et de glucides consistant essentiellement en une masse solide et homogène qui épouse sensiblement la forme de son récipient, comprenant la mise en contact d'un constituant carné, d'un constituant de type grain et d'un constituant de type sauce chauffée pendant une durée et à une température suffisantes pour préparer une composition alimentaire constituée essentiellement d'une masse solide et homogène, dans lequel la composition comprend au moins 65 % en poids de viande, et dans lequel le constituant carné n'est pas chauffé avant d'être mis en contact avec la sauce chauffée.

2. Procédé selon la revendication 1, dans lequel le constituant de type sauce chauffée est à une température de 65 à 85 °C avant la mise en contact du constituant carné ou du constituant de type grain.

3. Procédé selon la revendication 1, dans lequel le constituant carné comprend une ou plusieurs sources de viande choisies dans le groupe constitué par le muscle animal, la chair de squelette animal, les sous-produits animaux et les mélanges de muscle, de chair de squelette et de sous-produits.

4. Procédé selon la revendication 1, dans lequel le constituant de type grain comprend un ou plusieurs grains choisis dans le groupe constitué par la fibre d'avoine, la cellulose, la coque d'arachide, la pulpe de betterave, le riz étuvé, la fécule de maïs, la farine de gluten de maïs et les mélanges de ceux-ci.

5. Procédé selon la revendication 1, dans lequel la sauce chauffée comprend de l'eau et un ou plusieurs agents épaississants choisis dans le groupe constitué par la fécule de maïs, la gomme de guar, le glucose, le saccharose, un sirop de maïs à teneur élevée en fructose et les mélanges de ceux-ci.

6. Procédé selon la revendication 1, dans lequel la composition comprend au moins 75 % en poids de viande et de glucide non fibreux en une quantité inférieure à 15 % en poids de la composition.

7. Procédé selon la revendication 6, comprenant un glucide non fibreux en une quantité inférieure à 10 % en poids de la composition,

8. Procédé selon la revendication 6, dans lequel le constituant de type grain comprend de 2 % à 10 % en poids d'un mélange de grains comprenant un ou plusieurs grains.

9. Procédé selon la revendication 8, dans lequel le mélange de grains comprend un ou plusieurs grains choisis dans le groupe constitué par la fibre d'avoine, la cellulose, la coque d'arachide, la pulpe de betterave, le riz étuvé, la fécule de maïs, la farine de gluten de maïs et les mélanges de ceux-ci.

10. Procédé selon la revendication 1 comprenant de 5 % à 25 % en poids du constituant de type sauce chauffée.

11. Procédé selon la revendication 10, dans lequel le constituant de type sauce chauffée comprend de l'eau et un ou plusieurs agents épaississants choisis dans le groupe constitué par la fécule de maïs, la gomme de guar, le glucose, le saccharose, un sirop de maïs à teneur élevée en fructose et les mélanges de ceux-ci.

12. Procédé selon la revendication 1, dans lequel la viande comprend une ou plusieurs sources de viande choisies dans le groupe constitué par le muscle animal ; la chair de squelette animal ; les sous-produits animaux ; et les mélanges de muscle, de chair de squelette et de sous-produits.

13. Procédé selon la revendication 1, dans lequel la température du constituant carné est inférieure à 25 °C avant la mise en contact avec la sauce chauffée.
